# EUROPEAN PATENT APPLICATION

(11) **EP 2 052 712 A1**
(43) Date of publication of application: **29.04.2009**
(21) Application number: 07119353.6
(22) Date of filing: 26.10.2007
(51) Int. Cl.: A61K 6/00

(54) **Dental composition**

(71) Applicant: 3M Innovative Properties Company, St. Paul MN 55133-3427 (US)
(72) Inventor: Haeberlein, Ingo, 82362, Weilheim (DE); Kappler, Oliver, 82362, Weilheim (DE); Stoeger, Heidi, 86971, Peiting (DE); Hauke, Melanie, 82237, Wörthsee/Steinebach (DE); Luchterhandt, Thomas, 86926, Greifenberg (DE)
(74) Representative: Wallner, Astrid

(57) **Abstract**

The present invention relates to a bacteria detecting dental composition comprising a solution of at least one electron acceptor and at least one electron donor wherein the electron acceptor and the electron donor are selected that in the solution itself the electron donor can not transmit reduction equivalents to the electron acceptor and wherein the dental composition is substantially free of compounds that enable or facilitate the electron transmission between the electron acceptor and the electron donor.

The present invention further relates to the use of a composition comprising a solution of at least one electron acceptor and at least one electron donor wherein the electron acceptor and the electron donor are selected that in the solution itself the electron donor can not transmit reduction equivalents to the electron acceptor and wherein the composition is substantially free of compounds that enable or facilitate the electron transmission between the electron acceptor and the electron donor for site-directed intraoral detection of bacteria.

## Description

The present invention relates to a dental composition. It more specifically relates to the use of a composition for intra-oral site-directed detection of bacteria. It further relates to a method of intra-oral site-directed detection of bacteria.

### Background

Oral bacteria colonize hard and soft tissues in mouth such as gums, tongue, enamel, dentin, root cement and dental restorations. Oral bacteria residing on enamel, dentin, cement and dental restorations supra- and sub-gingival can cause demineralisation of the hard tissue and destruction of the periodontium.

Different technologies are available for detecting caries lesions. Most of these techniques involve detecting sites of demineralization that are associated with caries lesions. These technologies generally involve analyzing the degree of demineralization though any of a variety of techniques, including the use of visual and tactile methods, X-rays and electrochemical methods, and the use of certain dyes. The degree of observable demineralization determines how advanced the caries process has already damaged a tooth.

In general, technologies for detecting caries via detecting demineralization are not able to differentiate between infected and affected tooth tissue, which can lead to over-excavation of caries lesions. Affected tooth tissues, representing non-infected but partially demineralised hard tooth tissue, is found below the layer of infected hard tooth tissue. Infected hard tooth tissue should be removed but affected hard tooth tissue should be saved for remineralisation. For example, Ansari, Beeley, Reid and Foye (Journal of Oral Rehabilitation, 1999, 26; 453-458) determined that common dyes are not caries specific because they stain demineralized organic matrix and therefore cannot differentiate between infected non-remineralizable tissue and reversibly decalcified tissue.

Moreover, all procedures which detect caries via the degree of demineralization cannot immediately indicate if the caries process is still active or arrested. To differentiate between active and arrested caries lesions a second investigation, mostly many months later, is required to observe ongoing mineral loss over time.

Demineralization of hard tooth tissue is caused by the action of caries bacteria colonizing hard tooth tissues in mouth. All procedures which detect caries via the degree of demineralization have to wait until the caries bacteria have caused a detectable degree of mineral loss. In contrast, a procedure that is able to detect the deleterious action of caries bacteria on teeth before demineralization becomes detectable would have the advantage of being able to prevent initial caries even in its earliest stage.

US 3,332,743 disclose a test solution for evaluating individual's dental caries risk in measuring the color change of diazoresorcinol in saliva samples. However, this procedure does not provide any information about the location of caries bacteria on tooth surfaces.

In other procedures, plaque collected from tooth surfaces is used to evaluate the inherent activity to metabolize, glucose, saccharose or other sugars into organic acids outside the mouth. In one aspect, acid formation is followed by pH change occurring over time by pH indicators (US 4,359,455). In another aspect, the lactic acid formation in the plaque sample is evaluated by the use of enzymes (i.e. lactate dehydrogenase), whereby the enzymatic reaction is linked to redox dyes to generate a visual signal (DE 10108900). Although it is well-known that the caries activity in plaque differs significantly from site to site on teeth, both procedures mix plaque from several tooth surfaces to evaluate acid production in response to the presence of carbohydrates.

DE 101 08 900 discloses a process for the determination of individual's caries risk in presence of carbohydrates in saliva or plaque samples. Lactate dehydrogenase, Pyruvat, sucrose and oxidized nicotinamide adenine dinucleotide (NAD) are added and the formation of reduced nicotinamide adenine dinucleotide (NADH) is detected e.g. by redox indicators like MTT using an electron carrier, i.e. PMS.

US 4,351,899 and US 4,254,222 disclose procedures to measure lactic acid in biological fluids. Lactate dehydrogenase, NAD, an electron carrier and a tetrazolium dye as redox indicator is used to measure lactic acid. In case the biological fluid is saliva, an intra-oral site directed detection of caries bacteria residing on teeth/tooth surfaces is not possible.

JP 2004-113129 shows a liquid for specifying lactic acid producing region surrounding tooth surface, comprises preset amount of lactate dehydrogenase, oxidized nicotinamide adenine dinucleotide, electronic-transition agent, tetrazolium salt and water

EP 1191946 discloses a procedure for intra-oral directed detection of caries bacteria residing on teeth/tooth surfaces which is based on a dental impression material supplemented with lactate dehydrogenase, oxidized nicotinamide adenine dinucleotid, a redox dye, e.g. MTT and an electron carrier, e.g. PMS.

The [3-(4,5-dimethylthiazol-2-yl)-2, 5-diphenyltetrazolium bromide]-assay (MTT-Assay) is widely known as a test for the detection of living cells. With different modifications and adjustments the test includes an incubation of cells with MTT in an aqueous solution for usually 4 to 8 hours. E.g. Schweikl and Schmalz used it to determine the "Toxicity parameters for cytotoxicity testing of dental materials in two different mammalian cell lines" (Eur J Oral Sci. 1996 Jun; 104(3):292-9.).

Freimoser et al. published in "The MTT-Assay Is a Fast and Reliable Method for Colorimetric Determination of Fungal Cell Densities" (Applied And Environmental Microbiology 1999 August; 65(8): 3727-3729) the use of the MTT-Assay as a colorimetric method for the determination of cell densities. The samples containing the cells to be detected are incubated for 16 hours.

It would therefore be desirable to provide a composition, its use and a method for quick and reliable detection of the presence of bacteria in the mouth, on hard tooth tissue (enamel, root cement and dentin) and on dental restorations. It would further be desirable to provide a composition and a method for intra-oral site-directed detection of bacteria.

### Detail Description of the Invention

In one aspect, the invention provides a bacteria detecting dental composition comprising a solution of at least one electron acceptor and at least one electron donor. The electron acceptor and the electron donor are selected that in the solution itself the electron donor does not transmit reduction equivalents to the electron acceptor. Further, the dental composition is substantially free of compounds that enable or facilitate the electron transmission between the electron acceptor and the electron donor.

It is one main aspect of the present invention that the electron acceptor and the electron donor are selected that they can not react together in the solution. After applying the solution containing electron acceptor and electron donor to different sites of the oral cavity the electron acceptor becomes reduced due to the presence of bacteria.

It is very likely that the bacteria deliver a kind of electron carrier that facilitates the electron transmittal from the electron donor to the electron acceptor in the invented dental composition. Then, the reduction of the electron acceptor occurs by transfer of one or more electrons delivered by the electron donor when the invented dental composition gets in contact with bacteria.

The electron acceptor and/or the electron donor are selected that they have different colors in their reduced and in their oxidized state. For example, at least in its reduced state the electron acceptor shows a color in the visible range of about 380 nm to about 780 nm wave length. Thus, it is possible that the dental practitioner notices visually the color change of the electron acceptor between its oxidized and its reduced state in the composition.

There are different possibilities for this change. On the one hand the composition is colorless before applied to different intra-oral sites. Reduction of the electron acceptor causes a color change visibly by eye. On the other hand it could change that way, that the composition has a distinctive color before its application. If the electron acceptor is reduced the color can change to one showing a color which is visibly by eye and clearly distinctive from the color of the electron acceptor in the oxidized state.

In another embodiment of the present invention the electron acceptor in its reduced state emits fluorescence light in the visible wavelength range of about 380 nm to about 780 nm, which gives the electron acceptors in its reduced state a self-shining characteristic.

It is further possible for another embodiment of the present invention that electron donor is selected that it has different colors in its oxidized and in its reduced state. For example, the electron donor in its oxidized state shows a color in the visible range of about 380 nm to about 780 nm wave length. Thus, it is possible that the dental practitioner notices visually the color change of the electron donor between its reduced and its oxidized state in the composition.

The electron acceptor and the electron donor may be present each in a concentration that allows to persue the color change by eye. In one preferred embodiment, the electron donor is present in a concentration of about 0.5 to 13 mg/ml related to the total composition, more preferably of about 1.0 to about 3.5 mg/ml. In case the electron donor is one of the NADH- or NADPH-analogs as described in WO 98/33936, the concentration can be preferably about 10.0 to about 13.0 mg/ml. The electron acceptor may be present in a concentration of about 1.0 to about 3.5 mg/ml related to the total composition, preferably in a concentration of about 1.5 to about 2.5 mg/ml.

The solubility of the electron donor and the electron acceptor in the composition respectively the solvent of the inventive dental composition has to be that high that the intra-oral site-directed detection of bacteria is possible. That means that the concentration of the solved electron acceptor and the solved electron donor has to be as high as a distinctive color is perceivable. The solubility of the electron donor or the electron acceptor can be between 0.01 wt-% to 80 wt-% with relation to the total weight of the dental composition. Preferred is that the solubility of the electron acceptor and the electron donor is between 0.01 - 10 wt-% related to the total weight of the dental composition.

Preferably the electron acceptor shows different solubility in its oxidized and in its reduced state in the respective solvent. That is, when the electron acceptor gets reduced its solubility will be lowered that far that the electron acceptor in its reduced form precipitates at the site where the electron acceptor gets reduced, which allows intra-oral site directed detection of bacteria. In another embodiment the electron donor shows a lower solubility in its oxidized state in the respective solvent. Even here the solubility should diminish as much as a precipitation occurs at the site where the electron donor gets oxidized.

Of course there are other ways to make the detection of bacteria site-directed e.g. if the composition shows a high viscosity or forms a film.

As the inventive composition is a solution, it contains a solvent. This solvent can be water to form an aqueous solution of an electron acceptor and an electron donor. The solution further can contain as solvent an organic solvent or a mixture of organic solvents and/or water.

In the case of a mixture of one or more organic solvents with water the concentration of the organic solvents can be from about 0.1 wt.-% to about 60 wt.-% related to the total solution. Preferred as solvent is water. The one or more organic solvents may be used to adjust the solubility of the electron acceptor in its oxidized state and the electron donor in its reduced state in the solution or to improve the wettability of the area in the mouth the composition is to be applied to.

Suitable organic solvents are for example linear, branched or cyclic, saturated or unsaturated alcohols with 2 to about 10 C atoms, ketones as e.g. dialkyl ketones, esters, carboxylic acids, polymerizable substances of low viscosity such as polyethylene glycol (PEG), hydroxyethyl methacrylate or (2,3-epoxypropyl) methacrylate and mixtures of two or more of said types of solvents. Preferred solvents are water and water-alcohol mixtures.

Especially preferred alcoholic solvents are methanol, ethanol, iso-propanol and n-propanol.

Other suitable organic solvents are glycerin, dimethyl sulfoxide, tetrahydro furane, acetone, methyethyl ketone, cyclohexanol, toluene, methylen chloride, chloroform, alkanes and acetic acid alkyl esters, in particular acetic acid ethyl ester.

The pH value of the composition can be varied in a wide range. For example it is possible that the pH value is from about 1.0 to about 12.0. More preferred is a pH value of the composition from about 7.0 to about 10.0. If a two component system is used, the one component can have a pH value of about 10.0 to about12.0 and the other component can have a pH value of about 1.5 to about 4.0. The chosen pH should provide a condition where the electron acceptor and the electron donor show the oxidation and reduction properties as preferred. It further should be in the range that the color change properties and solubilities of electron acceptor and electron donor are as described above. The variation of the pH value of the composition may be made by selection of the type and the amount of any solvent or further ingredients as acids, e.g. HCl, acetic acid or the like.

The composition may also comprise a buffer. In the context of the present invention, all customary buffers are suitable, such as phosphate buffer, carbonate buffer, acetate buffer, formate buffer, citrate buffer, tris buffer, tris-(hydroxymethyl)-amino methane, glycylglycine buffer, glycine buffer, sodium phosphate buffer, sodium hydrogen phosphate buffer, sodium dihydrogen phosphate buffer, potassium phosphate buffer, potassium hydrogen phosphate buffer, potassium dihydrogen phosphate buffer or pyrophosphate buffer or mixtures thereof. Suitable are also sodium carbonate buffer, potassium carbonate buffer, sodium hydrogen carbonate buffer or potassium hydrogen carbonate buffer. Especially preferred as buffer are tris-(hydroxymethyl)-amino methane or glycylglycine.

The buffer concentration preferably lies in the range of about 0.01 to about 2.0 M.

The electron acceptor according to the present invention preferably is a tetrazolium compound or a tetrazolium derivative.

This tetrazolium compound or tetrazolium derivate can be selected e.g. from the group containing 2-(2'-benzothiazolyl)-5-styryl-3-(4'-phthalhydrazidyl)tetrazolium chloride, 2-(2-benzothiaolyl)-3-(4-nitrophenyl)-5-phenyltetrazolium bromide, 2-(2-benzothiaolyl)-3,5-diphenyltetrazolium bromide, 2-(4-iodophenyl)-3-(4-nitrophenyl)-5-phenyl-2H-tetrazolium chloride, 2-(4-iodophenyl)-3-(4-nitrophenyl)-5-phenyltetrazolium chloride, 2-(4-iodophenyl)-3-(4-nitrophenyl)-5-phenyltetrazolium chloride hydrate, 2,3,5-triphenyl-2H-tetrazolium chloride, 2,3,5-triphenyltetrazolium bromide, 2,3,5-triphenyltetrazolium chloride, 2,3,5-triphenyltetrazolium iodide, 2,3,5-tris(p-tolyl)tetrazolium chloride, 2,3-bis(3-chlorophenyl)-5-phenyltetrazolium chloride, 2,3-bis(3-fluorophenyl)-5-phenyltetrazolium chloride, 2,3-bis(3-methylphenyl)-5-phenyltetrazolium chloride, 2,3-bis(4-chlorophenyl)-5-phenyltetrazolium chloride, 2,3-bis(4-ethylphenyl)-5-phenyltetrazolium chloride, 2,3-bis(4-fluorophenyl)-5-phenyltetrazolium chloride, 2,3-bis(4-methoxyphenyl)-5-(4-cyanophenyl)tetrazolium chloride, 2,3-bis(4-methoxyphenyl)-5-phenyltetrazolium chloride, 2,3-bis(4-methylphenyl)-5-(4-cyanophenyl)tetrazolium chloride, 2,3-bis(4-nitrophenyl)-5-phenyltetrazolium chloride, 2,3-bis(4-nitrophenyl)-5-phenyltetrazolium chloride hydrate, 2,3-di(p-tolyl)-5-phenyltetrazolium chloride, 2,3-diphenyl-5-(2-thienyl)tetrazolium chloride, 2,3-diphenyl-5-(4-chlorophenyl)tetrazolium chloride, 2,3-diphenyl-5-(4-methoxyphenyl)tetrazolium chloride, 2,3-diphenyl-5-(p-diphenyl)tetrazolium chloride, 2,3-diphenyl-5-(p-tolyl)tetrazolium chloride, 2,3-diphenyl-5-aminotetrazolium chloride, 2,3-diphenyl-5-carboxytetrazolium chloride, 2,3-diphenyl-5-ethyltetrazolium chloride, 2,3-diphenyl-5-methyltetrazolium chloride, 2,5-di(p-tolyl)-3-phenyltetrazolium chloride, 2,5-diphenyl-3-(2,6-dimethylpheny)tetrazolium chloride, 2,5-diphenyl-3-(4-styrylphenyl)tetrazolium chloride, 2,5-diphenyl-3-(p-dipheny)tetrazolium chloride, 2-benzothiazolyl-3-(4-carboxy-2-methoxyphenyl-(5-[4-(2-sulfoethylcarbamoyl)phenyl]-2H-tetrazolium, 2-phenyl-3-(4-biphenylyl)-5-methyltetrazolium chloride, 2-phenyl-3-(4-carboxyphenyl)-5-methyltetrazolium chloride, 3-(3-nitrophenyl)-5-methyl-2-phenyltetrazolium chloride, 3-(4,5-dimethyl)-2-thiazolyl(-2,5-diphenyl-2H)-tetrazolium bromide, 3-(4,5-dimethyl)-2-thiazolyl(-2,5-diphenyl-2H)-tetrazolium chloride, 3-(4,5-dimethyl-2-thiazolyl)-2,5-diphenyltetrazolium bromide, 3-(4-nitrophenyl)-5-methyl-2-phenyltetrazolium chloride, 3,3'-(3,3'-dimethoxy-4,4'-diphenylene)bis(2-phenyl-5-veratryltetrazolium chloride), 3,3'-(3,3'-dimethoxy-4,4'-diphenylene)bis[2-(3-nitrophenyl)-5-phenyl]-2H-tetrazolium chloride, 4-[3-(4-iodophenyl)-2-(2,4-dinitrophenyl)-2H-5-tetrazolio]-1,3-benzene disulfonate, 4-[3-(4-iodophenyl)-2-(4-nitrophenyl)-2H-5-tetrazolio]-1,3-benzene disulfonate, 5-cyano-2,3-di-(p-tolyl)tetrazolium chloride, tetrazolium Blue chloride (Blue tetrazolium), cinnamyl nitro Blue tetrazolium chloride, lodonitrotetrazolium chloride, m-tolyltetrazolium Red, Neo-tetrazolium chloride diformazan, Nitro Blue Tetrazolium, Nitro Blue Tetrazolium monohydrate, Nitro Neotetrazolium chloride (Violet), o-tolyltetrazolium Red, p-anisyl Blue tetrazolium chloride, p-anisyl Blue tetrazolium chloride diformazan, piperonyl tetrazolium Blue, p-tolyltetrazolium Red, tetranitro Blue Tetrazolium, 2,5-diphenyl-3-(1-naphthyl)tetrazolium chloride (tetrazolium Violet), 2,2'-bipheny-4,4'-dihylbis-(3,5-diphenyl-2H-terazolium)-dichlorid (Tetrazolpurpur), thiocarbamyl nitro Blue tetrazolium chloride, veratryl tetrazolium Blue. Mixtures are also possible

Especially preferred electron acceptors are 2,3,5-triphenyltetrazolium bromide, 2,3,5-triphenyltetrazolium chloride, 2,3,5-triphenyltetrazolium iodide, 3-(4,5-Dimethyl)-2-thiazolyl(-2,5-diphenyl-2H)-tetrazolium bromide, Tetrazolium Violet/ 2,5-diphenyl-3-(1-naphthyl)tetrazolium chloride and/or Tetrazolpurpur/ 2,2'-bipheny-4,4'-dihylbis-(3,5-diphenyl-2H-terazolium)-dichloride.

For one preferred embodiment of the present invention the electron acceptor of the composition is 3-(4,5-dimethylthiazol-2-yl)-2,5-diphenyltetrazolium-bromide (MTT).

Differently to the compositions containing MTT known from the state of the art the present composition contains at least one electron donor. This electron donor can be selected from the group containing reduced nicotinamide adenine dinucleotide, reduced nicotinamide adenine dinucleotidephosphate, reduced flavine adenine dinucleotide, reduced flavine mononucleotide (riboflavine-5'-phosphate). Further possible is the use of a derivative of said donors or the use of NADPH and NADH analogs according to WO 98/33936 which is incorporated herein by reference and of mixtures of two or more donors of this group.

After exposing the inventive composition to bacteria, the transmittal of reduction equivalents and the above mentioned color change of the inventive composition occurs in 4 minutes or less, preferably in less than 2 minutes, more preferably in less than 1 minute, even more preferably in less than about 30 seconds and most preferably in about 20 seconds or less.

If the color change happens only after more than 4 minutes the composition is likely to be not appropriate for dental purposes as the dental practitioner and/or the patient will not wait for more than 4 minutes for a possible color change. In the dental practice it is important that procedures are fast because the patient and/or the dental practitioner will reduce waiting times as much as possible. Firstly to save costs and secondly to make the situation more convenient for the patient and the dental practitioner. Thus, as the compositions known from the art usually are incubated for 4 or 16 hours before the change of color is measured, the inventive composition includes a big progress.

The composition according to the present invention may optionally comprise further ingredients e.g. a quinone or a disulfide compound. One preferred quinone in the range of this invention is naphthoquinone. Preferred disulfide compounds in the range of this invention are oxidized lipoic acid, oxidized glutathione and/or cystine.

A quinone may be present in the inventive composition for example in a concentration of about 0.005 to about 0.5 mg/ml related to the total composition, preferably from about 0.01 to about 0.05 mg/ml.

Lipoic acid may be present in the inventive composition for example in a concentration of about 0.05 to about 2.0 mg/ml related to the total composition, preferably from about 0.1 to about 0.5 mg/ml.

One further embodiment of the described invention contains one or more auxiliary colorants. With an auxiliary colorant, the color of the inventive composition can be adjusted and the change of the color can be made better noticeable if necessary.

Thus, an appropriate auxiliary colorant can be a compound that does not change color when the electrons are transferred from the electron donor to the electron acceptor. Instead, the auxiliary colorant provides the composition of the invention with a first color that will improve detection of the color change of the electron acceptor. For example, the auxiliary colorant can provide a background color to enhance the appearance of the change in the absorption profile.

For example, auxiliary colorants in the composition include but are not limited to:
such as Patent Blue V, Patent Blue E 131, Erioglaucine, NEOZAPON blue 807 (commercially available from BASF, Ludwigshafen, Germany), methylene blue (commercially available from Hoechst), indigo carmine, indigo cartine (E 132), anthocyan (E 163); kurkumin (E 100), lactoflavin (E 101), Tatrazin (E 102), chinolin yellow (E 104), yellow orange S (E 110); cohenille (e 120), azorubin (E 122), amaranth (E 123), erythrosine (E 127), allura red (E 129) and ruby pigments (E 180). Typically, amounts of auxiliary colorant of from 5 ppm to 1 weight-%, preferably from 10 ppm to 1000 ppm and most preferably from 50 ppm to 500 ppm, can be used to prepare the compositions of the invention.

In another embodiment of the present invention the auxiliary colorant useful in the composition can be a fluorescence dye, which emits fluorescence light at a wavelength which is absorbed by the reduced form of the electron acceptor but is not absorbed by any other constituent of the invented dental composition. In accordance to the invention the fluorescence light emitted should have a wavelength of about 380 nm to about 780 nm. A fluorescent dye can be for example a rhodamine, such as preferably rhodamine 110.

A rhodamine may be present in the inventive composition for example in a concentration of about 0.005 to about 0.5 mg/ml related to the total composition, preferably from about 0.01 to about 0.2 mg/ml.

The composition of the invention may further comprise one or more additives such as rheology modifiers, stabilizers, fillers and the like. It further can contain one or more additives to adjust the pH value of the composition. The rheology modifiers thicken the composition so that it remains on the surface of the tooth. Rheology modifiers may include, for example, inorganic materials such as fumed silica, quartz and glass having particle sizes of less than 1 µm. In particular materials having particle sizes in the range of 10 to 500 nm are desirable. Rheology modifiers can also include organic materials capable of thickening aqueous solutions, such as poly(meth)acrylic acid and its salts; polysaccharides such as gum arabic and cellulose derivatives such as hydroxyethylcelluloses, carboxymethyl celluloses; hydroxyethyl starches, xanthanates and alginates.

Generally, the compositions of the invention comprise additives in the amount of 0 weight-% to 30 weight-%, preferably from 2 weight-% to 15 weight-% and most preferably from 3 weight-% to 13 weight-% based on total composition weight.

Other, optional additives can include stabilizers as preservatives such as benzoic acids, benzoic acid esters usually in an amount of 50 ppm to 5 weight-%.

Furthermore fillers such as glasses, quartz and Zirconica silicates, with a particle size of above 0.6 µm can be added to the composition. These filler may be present in an amount of 0 weight-% to 70 weight-%, preferably from 5 weight-% to 40 weight-% and most preferably from 10 weight-% to 40 weight-%.

The dental composition according to the invention can be provided as a one component system. Thus, all compounds are in one solution. The solution can be packed in different appropriate packaging device, e.g. a bottle, preferred a plastic material bottle of about 0.1 to 10 ml volume, a unit-dose-packaging of about 0.01 to about 1.0 ml volume or a bottle with integrated brush.

It further can be provided as a two or more component system.

The inventive composition comprising a solution of at least one electron acceptor and at least one electron donor wherein the electron acceptor and the electron donor are selected that in the solution itself the electron donor can not transmit reduction equivalents to the electron acceptor and wherein the composition is substantially free of compounds that enable or facilitate the electron transmission between the electron acceptor and the electron donor can be used particularly for site-directed intra-oral detection of bacteria.

The composition of the invention can be used for the site-directed intra-oral detection of bacteria on gum, enamel, dentin, root cement and dental restorations. The intra-oral applicability is a strong advantage of the composition as many other tests known from the art demand to collect a sample, e.g. of saliva, and to execute the detection of bacteria in vitro, in a separate vessel. Further, the composition and the use of this composition allow the site-directed detection of bacteria. By contrast many known tests only give advice about the presence of bacteria in the oral cavity. The invention described herein in view of its intra-oral application can provide a site-directed detection. Thus, additional information about the location of the bacteria is available without spending additional time.

A possible method of use of the dental composition comprises the steps of
a) providing a composition comprising a solution of at least one electron acceptor and at least one electron donor wherein the electron acceptor and the electron donor are selected that in the solution itself the electron donor can not transmit reduction equivalents to the electron acceptor and wherein the composition is substantially free of compounds that enable or facilitate the electron transmission between the electron acceptor and the electron donor,
b) applying said composition to gum, tongue, enamel, dentin, root cement and dental restorations, and
c) waiting for about 4 minutes or less.

A color change of the composition within this time of about 4 minute or less indicates that bacteria are detected. If no color change occurs it is a hint for the absence of bacteria especially for the absence of certain kind of bacteria. For example it is a hint for the absence of bacteria that produce a reducing environment. After detecting the color change the dental practitioner has to make his diagnosis if the bacteria are caries causing or not. Thus, the composition, the use of this composition and a method of use of this composition only describe a detection of bacteria and no information is implemented if these bacteria are causing illness or not. The detection of the bacteria itself is intra-oral and site-directed.

The advantage of the inventive composition and the use of this composition is that it is a quick time for the dental practitioner to wait until he can make a diagnosis.

In addition to the detection of bacteria simply on the surface of a tooth, the composition and the use of this composition is further applicable for the detection of bacteria in excavated caries lesions. Moreover, the dental practitioner can use the composition according to the invention also to detect bacteria after an excavation treatment has been carried out.

Preferably, inventive dental composition may be used to detect bacteria intra-orally on certain specific sites. For example, the detection of bacteria in margins of fillings, in margins of crown and bridges, white spots, pits and fissures, plaque, dental calculus and/or initial caries is possible.

The present invention also relates to a method of producing a dental composition comprising the steps of preparing a solution by dissolving at least one electron acceptor and at least one electron donor wherein the electron acceptor and the electron donor are selected that in the solution itself the electron donor can not transmit reduction equivalents to the electron acceptor and wherein the composition does not contain added compounds to enable or to facilitate the electron transmittal between said electron acceptor and said electron donor.

The composition of the above described invention can be used to manufacture a remedy for site-directed intra-oral detection of bacteria.

### For all of the following examples, the compounds used are defined as follows:

- Na₂-NADH:: reduced Nicotinamide adenine dinucleotide, disodium salt
- Acetyl-NADH:: reduced 3-Acetylpyridine Adenine Dinucleotide
- Tris:: Tris-(hydroxymethyl)-amino methane
- MTT:: 3-(4,5-dimethylthiazol-2yl)-2,5-diphenyltetrazolium-bromide
- DMSO:: Dimethylsulfoxide
- PMS:: phenazine methosulphate

### Example 1

### Component 1:

An aqueous solution was prepared by combining 100 ml of deionised water with 0.14 g β-Nicotinamide adenine dinucleotide, reduced disodium salt (Na₂-NADH) and 1.22 g Tris-(hydroxymethyl)-amino methane (Tris) adjusted to pH 11.4.

### Component 2:

A second solution was prepared of deionised water with 1.0 g 3-(4,5-dimethylthiazol-2yl)-2,5-diphenyltetrazolium-bromide (MTT) and 0.15 g KCl. This solution was then adjusted with HCl to a pH of 2.5. Deionised water was added until a total volume of 100 ml.
Example 1: Components 1 and 2 were mixed in a ratio of 3.6 to 1.

### Example 1a

### Component 1:

An aqueous solution was prepared by combining 100 ml of deionised water with 1.4 g Acetyl- NADH and 1.22 g Tris-(hydroxymethyl)-amino methane (Tris) adjusted to pH 11.4.

### Component 2:

A second solution was prepared of deionised water with 1.0 g 3-(4,5-dimethylthiazol-2yl)-2,5-diphenyltetrazolium-bromide (MTT) and 0.15 g KCl. This solution was then adjusted with HCl to a pH of 2.5. Deionised water was added until a total volume of 100 ml.
Example 1a: Components 1 and 2 were mixed in a ratio of 3.6 to 1.

Into a carious lesion of an extracted human tooth 1-3 drops of Example 1or Example 1a were given (depending on the size of the cavity). After 15 seconds, a blue-violet spot became visible formed by precipitated dye. Thus, clear distinct signals were observable indicating sites with elevated number of living bacteria.

### Example 2

### Component 1:

An aqueous solution was prepared by combining 100 ml of deionised water with 0.14 g Na₂-NADH and 1.22 g Tris adjusted to pH 11.4.

### Component 2:

A solution was prepared of deionised water with 1.0 g 3-(4,5-dimethylthiazol-2yl)-2,5-diphenyltetrazolium-bromide (MTT) and 0.15 g KCl. The solution was then adjusted with HCl to a pH of 2.5. Deionised water was added until a total volume of 100 ml.

### Component 3:

A solution was prepared by combining 1 ml Poly(ethyleneglycol) 200 with 1.00 mg 3-methyl-naphthoquinone.
Mixture 1: 5 ml of Component 2 are mixed with 1 ml of Component 3.
Example 2: Mixture 1 and Component 1 were mixed in a ratio of 1.0 to 3.6.

Into a carious lesion of an extracted human tooth 1-3 drops of Example 2 were given (depending on the size of the cavity). After 15 seconds, a blue-violet spot became visible formed by precipitated dye. Thus, clear distinct signals were observable indicating sites with elevated number of living bacteria.

### Example 3

### Component 1:

An aqueous solution was prepared by combining 1 ml of deionised water with 20 mg Na₂-NADH.

### Component 2:

2.00 mg Rhodamine was dissolved in 1.00 ml DMSO.

### Component 3:

13.214 g Glycylglycine was dissolved in deionised water. The solution was then adjusted with NaOH to a pH of 11.4. Deionised water was added until a total volume of 500 ml.

### Component 4:

A solution was prepared of deionised water with 1.0 g MTT and 0.15 g KCl. The solution was then adjusted with HCl to a pH of 2.5. Deionised water was added until a total volume of 100 ml.

### Component 5:

A solution was prepared by combining 1 ml Poly(ethyleneglycol) 200 with 1.00 mg 3-methyl-naphthoquinone.

### Mixture 1:

5 ml of component 4 are mixed with 1 ml of component 5.
Example 3a: The mixing ratio for Component 1, 2, 3 and Mixture 1 was 4.0 to 1.0 to 17.0 to 6.0.

Into a carious lesion of an extracted human tooth 1-3 drops of Example 3 were given (depending on the size of the cavity). After 10 seconds, a blue-violet spot became visible formed by precipitated dye. Thus, clear distinct signals were observable indicating sites with elevated number of living bacteria.

### Example 3a

### Component 1:

An aqueous solution was prepared by combining 1 ml of deionised water with 20 mg Na₂-NADH.

### Component 2:

2.00 mg Rhodamine was dissolved in 1.00 ml DMSO.

### Component 3:

13.214 g Glycylglycine was dissolved in deionised water. The solution was then adjusted with NaOH to a pH of 11.4. Deionised water was added until a total volume of 500 ml.

### Component 4:

A solution was prepared of deionised water with 1.0 g MTT and 0.15 g KCl. The solution was then adjusted with HCl to a pH of 2.5. Deionised water was added until a total volume of 100 ml.

### Mixture 1:

A solution was prepared by combining 1 ml Poly(ethyleneglycol) 200 with 1.00 mg 3-methyl-naphthoquinone.

### Mixture 1:

5 ml of component 3 are mixed with 1 ml of component 4.
Example 3: The mixing ratio for Component 1, 2, 3 and Mixture 1 was 4.0 to 1.0 to 17.0 to 6.0.

Into a carious lesion of an extracted human tooth 1-3 drops of Example 3 were given (depending on the size of the cavity). After 10 seconds, a blue-violet spot became visible formed by precipitated dye. Thus, clear distinct signals were observable indicating sites with elevated number of living bacteria.

### Example 4

### Component 1:

An aqueous solution was prepared by combining 1 ml of deionised water with 20 mg Na₂-NADH.

### Component 2:

10.00 mg of oxidized lipoic acid was dissolved in 1.00 ml Ethanol. Component 3:
13.214 g Glycylglycine was dissolved in deionised water. The solution was then adjusted with NaOH to a pH of 11.4. Deionised water was added until a total volume of 500 ml.

### Component 4:

A solution was prepared in deionised water with 1.0 g MTT and 0.15 g KCl. The solution was then adjusted with HCl to a pH of 2.5. Deionised water was added until a total volume of 100 ml.
Example 4: The mixing ratio for Component 1, 2, 3 and 4 was 4.0 to 1.0 to 17.0 to 6.0.

Into a carious lesion of an extracted human tooth 1-3 drops of Example 4 were given (depending on the size of the cavity). After 15 seconds, a blue-violet spot became visible formed by precipitated dye. Thus, clear distinct signals were observable indicating sites with elevated number of living bacteria.

### Example 5

### Component 1:

A solution was prepared of deionised water with 1.0 g MTT, 0.012 g PMS and 0.15 g KCl. The solution was then adjusted with HCl to a pH of 2.5. Deionised water was added until a total volume of 100 ml.

### Component 2:

13.214 g Glycylglycine was dissolved in deionised water. The solution was then adjusted with NaOH to a pH of 11.4. Deionised water was added until a total volume of 500 ml.
Example 5: The mixing ratio for Component 1 and 2 amounts 1.0 to 4.0.

Into a carious lesion of an extracted human tooth 1-3 drops of Example 5 were given (depending on the size of the cavity). After 120 seconds, a blue-violet spot became visible formed by precipitated dye. Thus, clear distinct signals were observable indicating sites with elevated number of living bacteria.

### Example 6

A solution was prepared of deionised water with 1.0 g MTT, 0.012 g PMS and 0.15 g KCl. The solution was then adjusted with HCl to a pH of 2.5. Deionised water was added until a total volume of 100 ml.

### Component 2:

13.214 g Glycylglycine was dissolved in deionised water. The solution was then adjusted with NaOH to a pH of 11.4. Deionised water was added until a total volume of 500 ml.

### Component 3:

2.00 mg Rhodamine was dissolved in 1.00 ml DMSO.
Example 6: The mixing ratio for Component 1, 2 and 3 amounts 25.0 to 100.0 to 1.0.

Into a carious lesion of an extracted human tooth 1-3 drops of Example 6 were given (depending on the size of the cavity). After 120 seconds, a blue-violet spot became visible formed by precipitated dye. Thus, clear distinct signals were observable indicating sites with elevated number of living bacteria.

### Example 7

### Component 1:

A solution was prepared of deionised water with 1.00 g MTT and 0.15 g KCl. The solution was then adjusted with HCl to a pH of 2.5. Deionised water was added until a total volume of 100 ml.

### Component 2:

An aqueous solution was prepared of deionised water with 1.22 g Tris. The solution was then adjusted with NaOH to a pH of 11.4. Deionised water was added until a total volume of 100 ml.
Example 7: Component 1 and Component 2 were mixed in a ratio of 1.0 to 3.6.

Into a carious lesion of an extracted human tooth 1-3 drops had been given (depending on the size of the cavity) of the Caries Indicator solution. After 300 seconds, a blue-violet spot became visible formed by precipitated dye. Thus, clear distinct signals were observable indicating sites with elevated number of living bacteria.

### Example 8

### Component 1:

A solution was prepared of deionised water with 1.00 g MTT and 0.15 g KCl. The solution was then adjusted with HCl to a pH of 2.5. Deionised water was added until a total volume of 100 ml.

### Component 2:

A solution was prepared by combining 380 mg Meldola's blue with 100 ml of deionised water.

### Mixture 1:

86.8 ml of Component 1 was mixed with 13.2 ml of Component 2.

### Component 3:

2.00 mg Rhodamine was dissolved in 1.00 ml DMSO.

### Component 4:

13.214 g Glycylglycine was dissolved in deionised water. The solution was then adjusted with NaOH to a pH of 11.4. Deionised water was added until a total volume of 500 ml.
Example 8: Mixture 1, Component 3 and 4 were mixed in a ratio of 25.0 to 1.0 to 100.0.

Into a carious lesion of an extracted human tooth 1-3 drops had been given (depending on the size of the cavity) of the Caries Indicator solution. After 300 seconds, a blue-violet spot became visible formed by precipitated dye. Thus, clear distinct signals were observable indicating sites with elevated number of living bacteria.

For all Examples 1 to 4 instead of Na₂-NADH different analogs of NADH and NADPH according to WO 98/33936 may be used. For example, in case of the analog 3-Acetylpyridine Adenine Dinucleotide in each of the Examples 1 to 4 an aqueous solution with an amount of 1.45 g Acetyl-NADH in 100 ml of deionised water and 0.12 mg Tris may be used. Other analogs of NADH and NADPH can be used also.

These results are outlined in the following table:

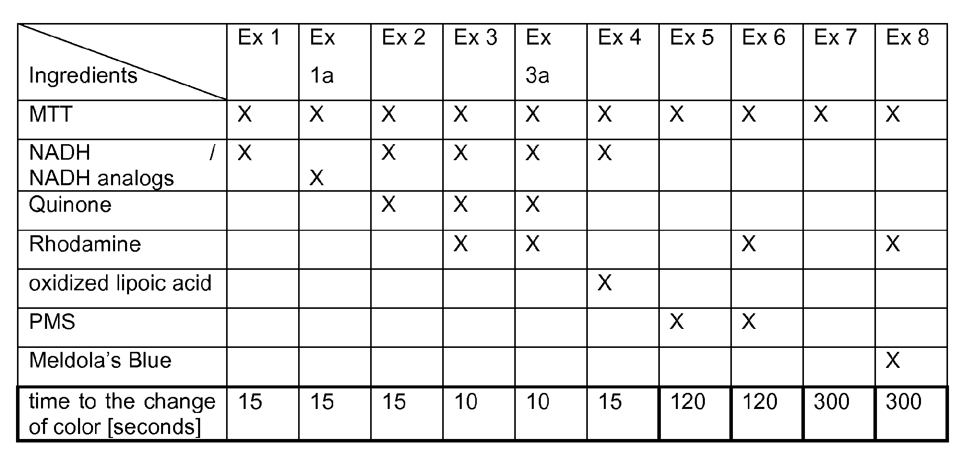

As can be seen from these examples the color change and the formation of a precipitation appeared much faster with the solutions containing an electron acceptor and an electron donor according to the invention. For all solutions without electron donor the time to the change of the color is significantly longer.

## Claims

1. A bacteria detecting dental composition comprising a solution of at least one electron acceptor and at least one electron donor wherein the electron acceptor and the electron donor are selected that in the solution itself the electron donor can not transmit reduction equivalents to the electron acceptor and wherein the dental composition is substantially free of compounds that enable or facilitate the electron transmission between the electron acceptor and the electron donor.

2. The dental composition according to claim1,
wherein the electron acceptor has a color in its reduced state that is different from the color it has in its oxidized state and has at least in its reduced state a perceivable color in the range of about 380 nm to about 780 nm wave length of the spectrum.

3. The dental composition according to claim 1,
wherein the electron acceptor in its reduced state has fluorescence emission properties in the range of about 380 nm to about 780 nm wave length.

4. The dental composition according to claim 1 to 3,
wherein the electron acceptor is a tetrazolium compound or a tetrazolium derivative.

5. The dental composition according to any of the preceding claims,
wherein the electron acceptor in its oxidized state has solubility of at least 0.01 wt.-% in the solution.

6. The dental composition according to any of the preceding claims,
wherein the electron acceptor in its reduced state precipitates from the solution.

7. The dental composition according to any of the preceding claims, containing the electron acceptor in a concentration of about 0.01 to 80 wt.-% with relation to the total weight of the dental composition.

8. The dental composition according to any of the preceding claims,
wherein the electron acceptor is selected from the group containing 2,3,5-triphenyltetrazolium bromide, 2,3,5-triphenyltetrazolium chloride, 2,3,5-triphenyltetrazolium iodide, 3-(4,5-dimethyl)-2-thiazolyl(-2,5-diphenyl-2H)-tetrazolium bromide, 2,5-diphenyl-3-(1-naphthyl)tetrazolium chloride (Tetrazolium Violet) and/or 2,2'-bipheny-4,4'-dihylbis-(3,5-diphenyl-2H-terazolium)-dichloride (Tetrazolpurpur), or mixtures thereof.

9. The dental composition according to any of the preceding claims,
wherein the electron acceptor is 3-(4,5-dimethylthiazol-2-yl)-2,5-diphenyltetrazolium-bromide (MTT).

10. The dental composition according to any of the preceding claims
wherein the electron donor is selected from the group consisting of reduced nicotinamide adenine dinucleotide, reduced nicotinamide adenine dinucleotidephosphate, reduced flavine adenine dinucleotide, reduced flavine mononucleotide or a derivative thereof and mixtures thereof.

11. The dental composition according to any of the preceding claims containing the electron donor in a concentration of about 0.01 to 80 wt.-% in relation to the total dental composition.

12. The dental composition according to any of the preceding claims
wherein the reduction equivalents are transmitted from the electron donor to the electron acceptor in the presence of bacteria within less than 4 minutes, preferably less than 2 minutes.

13. The dental composition according to any of the preceding claims
wherein the reduction equivalents are transmitted from the electron donor to the electron acceptor in the presence of bacteria within less than 30 seconds, preferably less than 20 seconds.

14. The dental composition according to any of the preceding claims, comprising a quinone, an oxidized glutathione, cystine and/or an oxidized lipoic acid.

15. The dental composition according to any of the preceding claims,
wherein the quinone is naphthoquinone.

16. The dental composition according to any of the preceding claims, comprising an auxiliary colorant.

17. The dental composition according to any of the preceding claims, comprising one or more additives selected from the group containing rheology modifiers, stabilizers, preservatives and fillers.

18. The dental composition according to any of the preceding claims,
wherein the composition is a two or more component system.

19. Use of a composition comprising a solution of at least one electron acceptor and at least one electron donor wherein the electron acceptor and the electron donor are selected that in the solution itself the electron donor can not transmit reduction equivalents to the electron acceptor and wherein the composition is substantially free of compounds that enable or facilitate the electron transmission between the electron acceptor and the electron donor for site-directed intra-oral detection of bacteria.

20. Use of a dental composition according to any of the claims 1 to 18 to manufacture a composition for the site-directed intra-oral detection of bacteria.

21. Use of a dental composition according to any of the claims 1 to 18 for site-directed intra-oral detection of bacteria.

22. Use of a dental composition according to any of the claims 1 to 18 for site-directed intra-oral detection of bacteria on gums, tongue, enamel, dentin, root cement, dental restorations, plaques and/or dental calculus.

23. A method for site-directed intra-oral detection of bacteria by use of a composition comprising a solution of at least one electron acceptor and at least one electron donor wherein the electron acceptor and the electron donor are selected that in the solution itself the electron donor can not transmit reduction equivalents to the electron acceptor and wherein the dental composition is substantially free of compounds that enable or facilitate the electron transmission between the electron acceptor and the electron donor.

24. A method for the detection of bacteria,
wherein the dental composition of any of the claims 1 to 18 is applied to gums, tongue, enamel, dentin, root cement, dental restorations, plaques and/or dental calculus.

25. A method according to claim 24 comprising the steps of:
a) providing a composition according to any of the claims 1 to 18,
b) applying said composition to gums, tongue, enamel, dentin, root cement, dental restorations, plaques and/or dental calculus.
